# EUROPEAN PATENT APPLICATION

(11) **EP 4 163 267 A1**
(43) Date of publication of application: **12.04.2023**
(21) Application number: 22782810.0
(22) Date of filing: 23.03.2022
(51) Int. Cl.: C07C 1/12, C07C 7/04, C07C 15/04, C07C 15/06, C07C 15/08, C07B 61/00, B01D 53/22

(54) **METHOD FOR PRODUCING AROMATIC COMPOUND**

(30) Priority: 21.07.2021 JP 2021120306
(71) Applicant: Chiyoda Corporation, Kanagawa 220-8765 (JP)
(72) Inventor: HIROHATA, Osamu, Yokohama-shi Kanagawa 2208765 (JP); WATANABE. Yuria, Yokohama-shi Kanagawa 2208765 (JP)
(74) Representative: Hartz, Nikolai
(86) International application number: PCT/JP2022/013664
(87) International publication number: WO 2023/002704

(57) **Abstract**

An object is to reduce an overall cost including the construction cost of a plant and its raw material and energy costs, and also reduce carbon dioxide emissions. An aromatic compound manufacturing method is characterized in that the method comprises: a step A of manufacturing an aromatic compound mixture from a raw material mixture gas containing carbon dioxide or carbon monoxide or both of these and hydrogen; a step B of manufacturing an aromatic compound mixture by a cracked petroleum-derived BTX manufacturing process, a reformate-derived BTX manufacturing process, and a synthetic BTX manufacturing process using naphtha as a raw material; a step C of combining the aromatic compound mixture manufactured in the step A and the aromatic compound mixture manufactured in the step B; and a step D of separating a desired aromatic compound from the aromatic compound mixture combined in the step C and purifying the desired aromatic compound.

## Description

### Technical Field

The present invention relates to a method of manufacturing an aromatic compound.

### Background Art

Aromatic compounds as represented by benzene, toluene, and xylene (hereinafter these and their derivatives will be collectively referred to as "BTX") are important chemical substances used in large quantities as basic raw materials in the petrochemical industry. BTX has originally been separated from crude light oil obtained by distilling tar generated as a result of dry distillation of coal (coal-derived BTX). At present, most of it has been replaced with petroleum-derived BTX manufactured using naphtha as a raw material. Petroleum-derived BTX includes: cracked petroleum-derived BTX obtained by separating BTX from by-product cracked petroleum produced as a result of manufacturing an olefin by pyrolysis of naphtha and purifying the BTX; reformate-derived BTX obtained by separating BTX from a reformate obtained by catalytic reforming of heavy naphtha and purifying the BTX; and synthetic BTX manufactured from light naphtha, LPG, and an olefin. Each of these methods uses a fossil resource (such as coal, petroleum, or natural gas, in particular, petroleum)-derived hydrocarbon (hereinafter referred to as "fossil resource-derived hydrocarbon") as a raw material, and therefore sustainability is a drawback due to the concerns about resource depletion in the future. This has led to researches for manufacturing hydrocarbons substantially equivalent to fossil resource-derived hydrocarbons (naphtha in particular) without using a fossil resource. An accomplishment of this is the development of techniques for manufacturing hydrocarbons equivalent to naphtha and LPG from a renewable biomass resource (hereinafter referred to as "biomass-derived hydrocarbon").

Incidentally, (a catalyst used in) a method of manufacturing BTX (in particular, para-xylene separated therefrom and purified) by using a so-called synthetic gas containing carbon monoxide and hydrogen as a raw material has been proposed (PTL 1) as a method of manufacturing an aromatic compound without using a fossil resource-derived hydrocarbon (or biomass-derived hydrocarbon) as a raw material. This method involves converting the synthetic gas into methanol with a catalyst having a ZnCr₂O₄spinel structure or the like, and then converting the methanol into a BTX mixture containing para-xylene with a catalyst being an H-ZSM-5 zeolite (hydrogen ZSM-5 zeolite) having some or all of its hydrogen atoms substituted (doped) with atoms of a metal such as zinc and having its outer surface coated with silicalite-1, or the like. Using a mixture of these catalysts to synthesize para-xylene from carbon monoxide and hydrogen by a single-stage reaction operation has also been proposed.

Moreover, there has been taught the possibility of single-stage synthesis of para-xylene by using a mixture gas of carbon dioxide and hydrogen as a raw material in lieu of a mixture gas of carbon monoxide and hydrogen (and a catalyst to be used in that synthesis) (PTL 2). In The method of PTL 2, a catalyst made of a chromium oxide is used as a methanol synthesis catalyst in lieu of the above spinel catalyst and the same H-ZSM-5 zeolite as the above (but not doped with zinc or the like) coated with silicalite-1 is used as a para-xylene synthesis catalyst, and a mixture of these methanol synthesis catalyst and para-xylene synthesis catalyst is used to synthesize para-xylene from carbon dioxide and hydrogen by a single-stage reaction operation. This method is considered to contribute to reducing carbon dioxide emissions since it not only consumes no fossil resource but also uses carbon dioxide as a raw material.

### Citation List

### Patent Literatures

PTL 1: Published Japanese Translation of PCT International Application No. 2020-535966
PTL 2: Japanese Patent Laid-Open No. 2019-205969

### Summary of Invention

### Technical Problem

The methods disclosed in PTL 1 and PTL 2 can be considered useful methods in the future with the concerns about fossil resource (petroleum in particular) depletion since they can synthesize BTX, or aromatic compounds, at high yield from a mixture gas of carbon monoxide or carbon dioxide and hydrogen without using a raw material made of a fossil resource (or biomass)-derived hydrocarbon, such as naphtha or LPG. However, in general, as compared to methods using a hydrocarbon such as naphtha or LPG, which is high in energy level, as a raw material, methods using carbon dioxide, which is low in energy level, as a raw material cannot be considered advantageous in terms of manufacturing cost. Thus, for these methods using carbon dioxide (or carbon monoxide manufactured from carbon dioxide by electroreduction or reverse shift reaction) as a raw material, it is desirable to utilize a part of an existing plant or construct a plant that manufactures BTX and chemical products derived therefrom through a process combining an existing plant so that, for example, the overall cost can be reduced and carbon dioxide emissions can be reduced. The present invention is aimed at achieving an object of providing a method that serves such a purpose.

### Solution to Problem

The present invention provides an aromatic compound manufacturing method characterized in that the method includes: a step A of manufacturing an aromatic compound mixture from a raw material mixture gas containing carbon dioxide or carbon monoxide or both of these and hydrogen; a step B of manufacturing an aromatic compound mixture from a raw material containing a fossil resource- or biomass-derived hydrocarbon; a step C of combining the aromatic compound mixture manufactured in the step A and the aromatic compound mixture manufactured in the step B; and a step D of separating a desired aromatic compound from the aromatic compound mixture combined in the step C and purifying the desired aromatic compound, thereby solves the above problem.

### Advantageous Effects of Invention

According to the method of the present invention, an existing plant may be utilized as equipment for performing the steps B and D. In this way, by simply adding only equipment for performing the steps A and C (and adjusting the existing equipment if necessary), a hybrid plant capable of performing all steps can be constructed (the plant is named this way since it is a combination of an existing BTX plant and a plant for manufacturing BTX from a raw material mixture gas containing carbon dioxide or carbon monoxide or both of these and hydrogen). Moreover, by utilizing an excess raw material produced in the step A or B and a substance to be discharged with each other, it is possible to reduce waste of substances and energy and manufacture an aromatic compound at low cost.

### Brief Description of Drawings

Fig. 1 illustrates an example of an apparatus configuration suitable for implementing a method of the present invention.
Fig. 2 illustrates an apparatus configuration used to implement an example of the method of the present invention.
Fig. 3 illustrates an apparatus configuration used to implement a comparative example of the method of the present invention.

### Description of Embodiments

A method according to the present invention includes: a step A of manufacturing an aromatic compound mixture from a raw material mixture gas containing carbon dioxide or carbon monoxide or both of these and hydrogen; a step B of manufacturing an aromatic compound mixture from a raw material containing a fossil resource- or biomass-derived hydrocarbon; a step C of combining the aromatic compound mixture manufactured in the step A and the aromatic compound mixture manufactured in the step B; and a step D of separating a desired aromatic compound from the aromatic compound mixture combined in the step C and purifying the desired aromatic compound.

### <Step A>

In the step A, an aromatic compound mixture is manufactured from a raw material mixture gas containing carbon dioxide or carbon monoxide or both of these and hydrogen. In the case of manufacturing a product containing aromatic compounds with a mixture gas of carbon monoxide and hydrogen (a so-called synthetic gas) as a raw material, carbon monoxide is hydrogenated, thereby producing methanol or dimethyl ether, as shown in (1). From the methanol or dimethyl ether thus produced, various aromatic compound mixtures are produced via lower olefins, as shown in Equation (2).

2CO + 2H₂ → 2CH₃OH (↔ CH₃OCH₃ + H₂O) (1)

CH₃OCH₃ → C₂H₄, C₃H₆, etc. → various aromatic compounds (2)

In this case, as a catalyst for causing the methanol synthesis reaction of Equation (1) to progress, a spinel structure catalyst made of a composite oxide of zinc (or copper) and chromium is preferably usable, as described in PTL 1. As a catalyst for causing the reaction of Equation (2) to progress to synthesize an aromatic compound mixture, a Zn/H-ZSM-5 zeolite (hydrogen ZSM-5 zeolite doped with zinc) is preferably usable. Here, by coating the outer surface of the Zn/H-ZSM-5 zeolite with a silicon-containing compound (preferably one having the same lattice structure as the ZSM-5 zeolite and having no acid site, such as silicalite-1), it is possible to increase the production ratio of para-xylene, which is a BTX having a particularly high demand. Note that, by using a mixture of these catalysts, the reaction of Equation (1) and the reaction of Equation (2) progress continuously or simultaneously. Accordingly, the product containing aromatic compounds can be manufactured with a single-stage reactor.

On the other hand, in the case of manufacturing a product containing aromatic compounds with a mixture gas of carbon dioxide and hydrogen as a raw material, a reaction that produces methanol or dimethyl ether progresses as shown in Equation (3).

CO₂ + 3H₂ → CH₃OH + H₂O (↔ CH₃OCH₃ + 2H₂O) (3)

In other words, a larger amount of by-product water is produced when methanol or dimethyl ether is produced. Thus, as described in PTL 2, in lieu of the above-mentioned catalyst made of a composite oxide of zinc (or copper) and chromium, a catalyst made of a chromium oxide (containing neither zinc nor copper) may be used as a catalyst for causing the reaction of Equation (3) to progress, and hydrogen H-ZSM-5 not doped with zinc may be used as the catalyst for causing the reaction of Equation (2) to progress. In this way, the yield of the aromatic compounds can be raised.

In sum, it suffices that the step A in the present invention uses a mixture of a combination of a catalyst containing an oxide of at least one metal selected as appropriate from among chromium, zinc, and copper, and a catalyst containing an H-ZSM-5 zeolite doped with zinc or the like as appropriate (covered with a silicon-containing compound, such as silicalite-1, if it is desired to increase the production ratio of para-xylene) according to the ratio of the carbon dioxide and carbon monoxide present in the raw material mixture gas and the contents of other components. Herein, a hydrogen ZSM-5 zeolite or a hydrogen ZSM-5 zeolite doped (ion-exchanged) with any of various ions will be collectively referred to as ZSM-5-type zeolite.

Note that, as will be described later, in the present invention, it is preferable to perform gas-liquid separation on a product containing the aromatic compound mixture obtained in the step A and combine only the separated oil phase (containing most of the aromatic compounds) with the aromatic compound mixture obtained in the step B. It is preferable to return the gas phase separated by the gas-liquid separation (containing unreacted carbon dioxide, carbon monoxide, and hydrogen) to the entrance side of the reactor. In that case, as the ratio of carbon dioxide and carbon monoxide and the contents of other components mentioned above, those at the entrance of the reactor (those of a combination of the raw material gas and the returned gas phase component) should be considered.

In the case of using carbon dioxide as at least a part of the raw material mixture gas in the step A, it is possible to utilize carbon dioxide separated from an exhaust gas from an apparatus at a thermal power plant or any of various heating furnaces that combusts a fuel which produces carbon dioxide, carbon dioxide separated in an ammonia manufacturing apparatus, an ethylene glycol manufacturing apparatus, or a hydrogen manufacturing apparatus, carbon dioxide separated from a gas produced by a coal, biomass, or waste gasification furnace, carbon dioxide separated from a blast furnace at an ironworks, carbon dioxide separated from the atmospheric air, and the like. Doing so reduces carbon dioxide emissions into the atmosphere and is therefore preferable.

As the hydrogen forming the raw material mixture gas, it is preferable to use hydrogen produced by electrolysis of water using renewable energy generated by photovoltaic, wind, water, geothermal, etc. power generation or electric power generated by nuclear power generation. Alternatively, if excess hydrogen is generated in the later-described step B, such excess hydrogen can be used as a part of the raw material mixture gas in the step A. Also, a synthetic gas produced by a gasification furnace, an off-gas discharged from a blast furnace at an ironworks, an off-gas separated in a hydrogen manufacturing apparatus, a synthetic gas produced by co-electrolysis of water and carbon dioxide, a synthetic gas produced by a reverse shift reaction of hydrogen and carbon dioxide, or the like may be used as the raw material mixture gas.

The reactor type is not particularly limited as long as it is capable of performing a gas-solid contact operation with the raw material mixture gas (gas) and a reaction catalyst (solid) and maintaining a desired temperature and pressure (such as a packed bed, a moving bed, or a fluidized bed). Nonetheless, a packed bed is preferable since it has good contact efficiency, is less prone to channeling, and causes less mechanical damage to the catalyst particles. With the packed bed type, while the amount of the catalyst to be packed and the gas flow rate can be set as appropriate, the catalyst to be packed and the gas flow rate are desirably set such that the superficial space velocity (SV) is about 100 to 10000/hr. Also, the reaction temperature is preferably set at about 250°C to 600°C, and the reaction pressure is preferably set at about 1 to 10 MPaG.

The gaseous reaction product taken out of the reactor is preferably cooled to condensate high-boiling components including the aromatic compounds, followed by gas-liquid separation into a gas phase and a liquid phase and further separation of the liquid phase into a water phase containing water and water-soluble components such as alcohol produced by the reaction and an oil phase containing the aromatic compounds and the like which are immiscible in water. Specifically, the cooled reaction product is separated into a water phase forming a lower layer, an oil phase forming a middle layer, and a gas phase forming an upper layer in this order from the bottom of the gas-liquid separator. Thus, each phase's fluid may be drawn out of the apparatus from the position where its layer is formed. Alternatively, the gas-liquid mixture may firstly be separated into a gas phase and a liquid phase, and the liquid phase may then be separated into an oil phase and a water phase by a separation method utilizing the difference in relative density, such as centrifugation or sedimentation.

When the reaction product is separated into the water phase, the oil phase, and the gas phase and taken out of the gas-liquid separator, almost all aromatic compounds including BTX are contained in the oil phase. Thus, in the later-described step C, only the oil phase may be combined with the aromatic compound mixture manufactured in the step B. The gas phase, on the other hand, contains carbon dioxide, carbon monoxide, and hydrogen, which are unreacted gases. Hence, it is preferable to circulate this to the reactor by returning it to the entrance side of a heater located before the reactor. Note that the gas phase not only contains these unreacted gases but also lower alkanes having one to four carbon atoms (mainly methane), which are by-products, and such lower alkanes are hardly involved in the aromatic compound synthesis reaction inside the reactor. Accordingly, these alkanes gradually accumulate in the circulation channel. It is therefore necessary to purge a part of the gas in the circulation channel to the outside. Purging about 1 to 20% by volume of the entire amount to be circulated can maintain the concentration of the lower alkanes in the gas in the circulation channel at less than 40% by volume. Note that the water phase does not contain many useful components, and it is therefore preferable to treat the water phase with a waste water treatment apparatus and then release it to the outside.

The gas purged from the circulation channel contains the unreacted carbon dioxide, carbon monoxide, and hydrogen and the by-product lower alkanes, and is therefore usable as a fuel gas for heating. However, it is preferable to separate the hydrogen contained in this purged gas by membrane separation, adsorptive separation (such as pressure swing adsorption), or the like to collect only the hydrogen from the purged gas and recycle it, in order to save hydrogen, which is necessary as a raw material gas. The carbon dioxide and carbon monoxide in the purged gas may also be collected in addition to the hydrogen. By performing membrane separation using an appropriate membrane, these gases can be separated and collected from the purged gas.

As for the heating of the raw material mixture gas performed at the entrance side of the reactor and the cooling of the produced gas mixture performed on the exit side of the reactor, the heat collected by the cooling of the produced gas mixture may be used to heat the raw material mixture gas. Doing so can save energy necessary for the heating and cooling and is therefore preferable. Moreover, if heat exchange alone is not sufficient to cool the produced gas mixture, the produced gas mixture that has been cooled to some extent by the heat exchange operation may be cooled further.

### <Step B>

In the step B, an aromatic compound mixture is manufactured from a raw material made of a fossil resource- or biomass-derived hydrocarbon. For example, the aromatic compound mixture is manufactured by a cracked petroleum-derived BTX manufacturing process, reformate-derived BTX manufacturing process, or synthetic BTX manufacturing process using naphtha, LPG, or the like as a raw material.

The cracked petroleum-based BTX manufacturing process is a process in which BTX contained in cracked petroleum generated as a by-product as a result of manufacturing an olefin via pyrolysis of naphtha is separated and taken out by distillation or the like. Since the cracked petroleum contains unsaturated compounds and sulfur compounds, it is preferable to perform a hydrogenation process and then perform the separation operation.

The reformate-derived BTX manufacturing process is a process in which heavy naphtha is catalytically reformed to cyclize and dehydrogenate the paraffins and naphthenes contained in the heavy naphtha for aromatization. By the catalytic reforming process, BTX is manufactured along with a high-octane gasoline base. There are various types of reformate-derived BTX manufacturing processes differing in the type of reaction tower and catalyst regeneration method, and there are various kinds of catalysts to be used. In the case of performing a reformate-derived BTX manufacturing process in the step B in the present invention, it may be performed by employing any of these processes or catalysts.

The synthetic BTX manufacturing process is a process in which an aromatic compound mixture is manufactured by aromatizing light naphtha, LPG, or an olefin into BTX. There are various types of processes such as one using light naphtha as a raw material, one using C4/C5 olefin as a raw material, and one using LPG or light naphtha as a raw material. In the case of performing a synthesizing process in the step B in the present invention, it may be performed by employing any of these processes,

Incidentally, excess hydrogen is generated in the catalytic reforming in the reformate-derived BTX manufacturing process and the synthetic BTX manufacturing process. In conventional practice, such excess hydrogen is used in the desulfurization of kerosene and light oil at oil factories and the like, yet there is still an excess, which is used as a fuel gas for heating and the like. In the future, the spread of biofuels, the shift to EVs, and so on due to the trend toward decarbonization will reduce the demand for petroleum-derived kerosene and light oil, which in turn will reduce the demand for hydrogen for desulfurization. Accordingly, there will be more excess by-product hydrogen generated by catalytic reforming. In the present invention, the excess hydrogen generated in the step B can be used as a part of the raw material mixture gas in the step A. This enables effective utilization of the excess hydrogen.

### <Step C>

In the step C, the aromatic compound mixture manufactured in the step A and the aromatic compound mixture manufactured in the step B are combined. The step A manufactures aromatic compounds from mixture gas raw materials, or carbon monoxide or carbon dioxide and hydrogen, and the step B manufactures aromatic compounds from a hydrocarbon raw material such as naphtha or LPG. Each of those products is a mixture of aromatic compounds including BTX and can therefore be separated and purified in basically the same purification step. Then, if there is an existing plant that performs the step B and a purification step after it, that purification step can be utilized to separate and purify the product of the step A along with the product of the step B.

In the step C, the type of the apparatus for combining the product of the step A and the product of the step B is not particularly limited. A flow of the product of the step A and a flow of the product of the step B may be caused to enter a single mixing tank and mixed inside the tank. Alternatively, a mechanism may be provided by which a flow of the product of the step A and a flow of the product of the step B are simply converged and they are mixed after being converged.

In the case of employing the reformate-derived BTX manufacturing process in the step B, the ratio of the xylenes or the heavy aromatic compounds having nine or more carbon atoms contained in the produced mixture is large. For this reason, before or after the produced mixture is combined with the product of the step A, these are preferably separated first. Also, in the case of employing the cracked petroleum-derived BTX manufacturing process (or a reformate-derived BTX manufacturing process including it) in the step B, the produced mixture contains large amounts of light paraffins having about six to seven carbon atoms. For this reason, before or after the produced mixture is combined with the product of the step A, these are preferably separated first. The above, however, does not apply to the case of employing only the synthetic BTX manufacturing process in the step B since the contents of heavy aromatic compounds and light paraffins are low.

### <Step D>

In the step D, desired aromatic compounds are separated from the aromatic compound mixture combined in the step C and purified. The mixture combined in the step C contains various aromatic compounds such as benzene, toluene, ortho-xylene, meta-xylene, para-xylene, ethylbenzene, and trimethylbenzene. Thus, these need to be separated into individual target compounds and purified. Specifically, it is preferable to firstly separate the obtained mixture by a distillation operation into benzene, toluene, and the like lower in boiling point than xylenes (ortho-xylene, meta-xylene, para-xylene) and ethylbenzene as a lower boiling fraction and into trimethylbenzene and the like higher in boiling point than xylenes and ethylbenzene as a higher boiling fraction.

The xylenes (ortho-xylene, meta-xylene, para-xylene) and ethylbenzene obtained as a result of the separative removal of the lower boiling fraction and the higher boiling fraction have boiling points close to one another. Thus, separating these only by a distillation operation is insufficient. It is therefore preferable to obtain these mixtures as a xylene fraction and then adsorb and separate this mixture with a zeolite. The zeolite has pores with the molecular size of para-xylene, and therefore well adsorbs para-xylene but hardly adsorbs ortho-xylene, meta-xylene, or ethylbenzene, and functions as a molecular sieve. That is, components other than para-xylene (ortho-xylene, meta-xylene, ethylbenzene, and other impurities) are not adsorbed by the zeolite and pass through the adsorption tower. Hence, by repeating adsorption and desorption of the mixture by using the zeolite, para-xylene can be concentrated and purified. Specifically, highly-concentrated para-xylene can be obtained by causing the xylene mixture to flow through an adsorption tower filled with an adsorbent (zeolite) to thereby adsorb only para-xylene, bringing a desorbent into contact with the adsorbent containing the para-xylene to thereby desorb the para-xylene, and separating the desorbent and the para-xylene mixture in a distillation tower.

Regarding the lower boiling fraction, the light paraffins can be removed by aromatic compound extractive separation, and benzene can be separated from toluene (and small amounts of xylenes) and purified by a distillation operation. Note that toluene can be converted into benzene by dealkylation and also converted into benzene and xylenes by disproportionation (transalkylation) . On the other hand, the heavy aromatic compounds separated as the higher boiling fraction can be utilized as additives for high-octane gasoline. Of these, trimethylbenzene can be mixed in toluene and subjected to a disproportionation process to be partly converted into a xylene mixture containing para-xylene, and then returned to the entrance side of the purification step. Specifically, the disproportionation process is performed by heating the mixture containing toluene and trimethylbenzene and passing the mixture through a reactor filled with a zeolite catalyst.

Moreover, a xylene isomerization process may be performed as necessary. The ortho-xylene and meta-xylene remaining after the high-purity para-xylene is obtained in the purification step can be partly converted into para-xylene by an isomerization process and returned to the entrance side of the purification step. Specifically, the isomerization process is performed by heating the mixture of the ortho-xylene and meta-xylene after the para-xylene separation and passing the mixture through a reactor filled with a zeolite catalyst.

### [Examples]

### Example 1

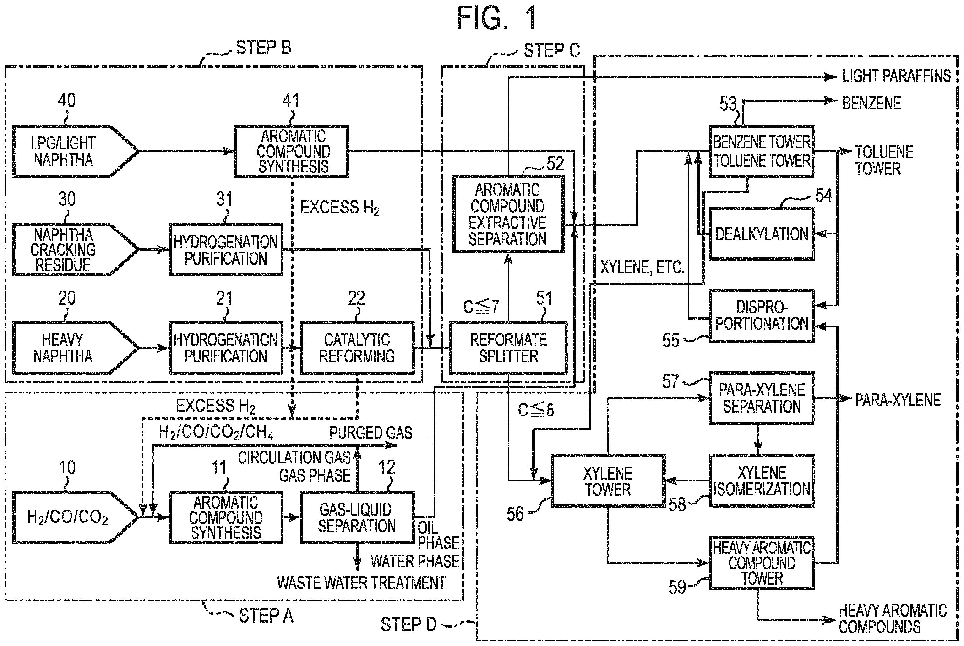

Fig. 1 illustrates an example process flow of the aromatic compound manufacturing method of the present invention in which the step A includes a step of manufacturing an aromatic compound mixture from a raw material mixture gas 10 containing hydrogen, carbon monoxide, and carbon dioxide, and the step B includes one or a combination of two of more of a step of manufacturing an aromatic compound mixture by catalytically reforming heavy naphtha 20 (reformate-derived BTX manufacturing process), a step of separating aromatic compounds from a thermal cracking residue 30 generated as a result of manufacturing an olefin from naphtha (cracked petroleum-derived BTX manufacturing process), and a step of manufacturing an aromatic compound mixture by aromatizing light naphtha or LPG 40 (synthetic BTX manufacturing process).

### (Step A)

In Fig. 1, the raw material mixture gas 10 containing hydrogen, carbon monoxide, and carbon dioxide is heated and introduced into a reactor 11 for synthesizing aromatic compounds. Inside the reactor 11, a catalyst containing an oxide of at least one metal selected from among chromium, zinc, and copper and a catalyst containing a ZSM-5-type zeolite are mixed and filled, thereby forming a mixed catalyst layer. By contacting the mixed catalyst under a high-temperature high-pressure atmosphere at 250°C to 600°C and 1 to 10 MPaG inside the reactor 11, the raw material mixture gas gets reacted and becomes a produced gas mixture containing various aromatic compounds. The produced gas mixture thus obtained is cooled to around normal temperature and introduced into a gas-liquid-liquid separator 12. Inside the gas-liquid-liquid separator 12, the produced gas mixture, containing condensed high-boiling components, is separated into three layers, namely, a water phase (lower layer) containing water-soluble components, an oil phase (middle layer) containing an aromatic compound mixture, and a gas phase (upper layer) containing unreacted gases.

The oil phase forming the middle layer containing an aromatic compound mixture among the three layers is drawn out of the gas-liquid-liquid separator 12 and then combined with the aromatic compound mixture manufactured in the step B (step C), followed by separation into various aromatic compound products and purification thereof (step D).

The gas phase forming the upper layer contains unreacted gases such as hydrogen, carbon dioxide, and carbon monoxide and lower alkanes, which are by-products. Thus, the gas phase is drawn out of the gas-liquid-liquid separator 12, mixed as a circulation gas into the flow of the raw material mixture gas on the entrance side of the reactor 11, re-heated, and subjected to the aromatic compound synthesis reaction. Note that a part of the circulation gas is purged to the outside of the system in order to prevent accumulation of the lower alkanes and the like and effectively utilized as a fuel gas for the heat source of a heating furnace or the like at a nearby location.

The water phase forming the lower layer is drawn out of the gas-liquid-liquid separator 12, sent to and treated by a waste water treatment apparatus to remove the water-soluble organic substances and the like, and then discharged to the outside of the system.

### (Step B)

In Fig. 1, a hydrogenation purification process 21 is performed to remove sulfur components and the like from the heavy naphtha 20, which is then introduced into a reformer 22 filled with a reforming catalyst and catalytically reformed under a high-temperature atmosphere at about 500°C and 0.4 MPaG to thereby be converted into a mixture (reformate) containing various aromatic compounds. Also, a hydrogenation purification process 31 is performed to remove sulfur components and the like from the naphtha cracking residue 30, which is then combined as a mixture containing various aromatic compounds with the reformate (step C). Also, the light naphtha or LPG 40 is aromatized by an aromatization synthesis process 41 under a high-temperature atmosphere at about 500°C and 0.5 MPaG in the presence of a catalyst, and thereby becomes a mixture containing various aromatic compounds.

### (Step C)

In Fig. 1, the oil phase drawn out of the middle layer in the gas-liquid-liquid separator 12 in the step A and the product (reformate) obtained by the hydrogenation purification 21 and the catalytic reforming 22 of the heavy naphtha 20 and the product obtained by the hydrogenation purification of the naphtha cracking residue 30 in the step B are each a mixture of aromatic compounds including benzene, toluene, and xylene (BTX). Thus, after these are combined, they are separated by a reformate splitter 51 into light components having seven or less carbon atoms such as benzene and toluene and heavy components having eight or more carbon atoms such as xylene and ethylbenzene. Thereafter, of the light components, paraffins having boiling points close to those of benzene and toluene are separated and removed by an aromatic compound extraction separator 52.

On the other hand, the product obtained by aromatizing light naphtha or LPG in the step B is mainly made of benzene and toluene and also small amounts of xylenes but hardly contains paraffins having boiling points close to those of benzene and toluene. For this reason, the product is converged into the flow of the light components after the removal of these by the aromatic compound extraction separator 52.

### Step D)

The aromatic compound mixture combined in the step C is then separated into individual aromatic compounds in a usual manner by operations such as distillation, adsorption, and extraction. In Fig. 1, the light components having seven or less carbon atoms separated by the reformate splitter 51 are separated into benzene, toluene, a small amount of xylene contained, and so on through distillation towers (benzene tower, toluene tower) 53 after the separative removal of the light paraffins by the aromatic compound extractive separator 52. Compared to the ratio of benzene and toluene produced, the ratio of demand for both is greater for benzene. Thus, the ratio of the amounts to be produced can be adjusted to match the demand by, for example, dealkylation 54 of a part of the produced toluene to convert it into benzene and disproportionation 55 of the benzene and xylene.

On the other hand, the compounds having eight or more carbon atoms separated by the reformate splitter 51 or the toluene tower are separated into xylene and heavy aromatic compounds having nine or more carbon atoms by a distillation tower (xylene tower) 56. Since the three kinds of xylene, or ortho-, meta-, and paraxylenes, have close boiling points, it is difficult to separate them by distillation. Thus, para-xylene, which has the highest demand among these three kinds of mixtures, is separated by adsorption 57. Moreover, the remaining two kinds of xylene can also be converted into para-xylene by a xylene isomerization process 58. Note that, of the heavy aromatic compounds separated from the xylene, a part of the component having eight carbon atoms is subjected to the disproportionation process 55 through a heavy aromatic compound column 59 while the other part is used as a gasoline additive or the like for increasing the octane rating.

In Fig. 1, excess hydrogen is generated in the process of manufacturing an aromatic compound mixture (reformate) by the catalytic reforming 22 of the heavy naphtha 20 after its hydrogenation purification and manufacturing an aromatic compound mixture from the light naphtha or LPG 40 by the synthetic aromatic compound manufacturing process 41. This is used as a raw material for synthesizing aromatic compounds from the hydrogen, carbon monoxide, and carbon dioxide 10. This enables effective utilization of the excess hydrogen.

### Example 2

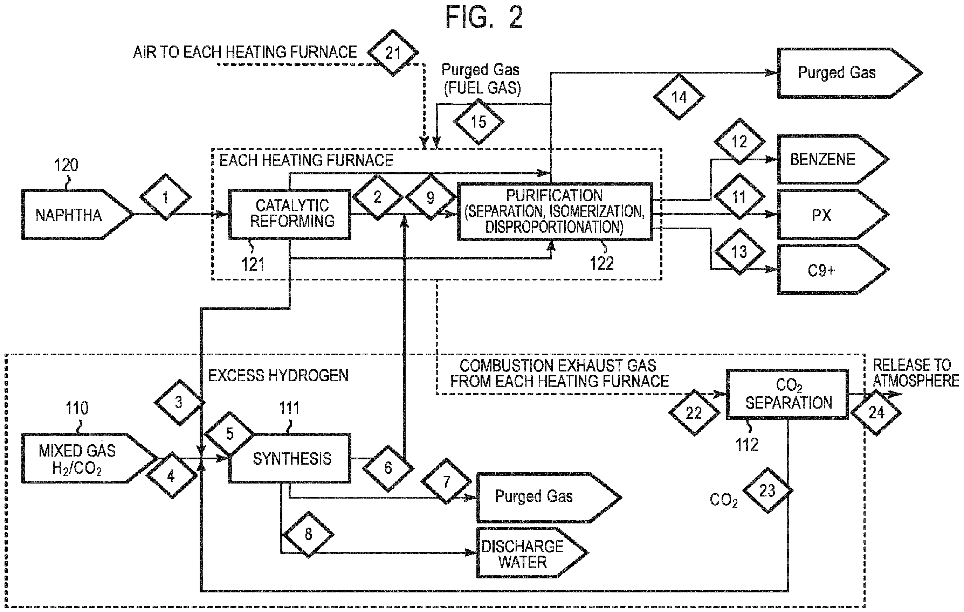

Fig. 2 illustrates an example process configuration of the aromatic compound manufacturing method of the present invention in which the step A includes a step of manufacturing an aromatic compound mixture from a raw material mixture gas containing hydrogen and carbon dioxide, and the step B includes only a step of manufacturing an aromatic compound mixture by catalytically reforming heavy naphtha (reformate-derived BTX manufacturing process).

### (Step A)

In Fig. 2, a raw material mixture gas 110 containing hydrogen and carbon dioxide is heated and introduced into a reactor 111 for synthesizing aromatic compounds. Inside the reactor 111, a catalyst containing an oxide of at least one metal selected from among chromium, zinc, and copper and a catalyst containing a ZSM-5-type zeolite with its surface coated with silicalite-1 are mixed and filled, thereby forming a mixed catalyst layer. By contacting the mixed catalyst under a high-temperature high-pressure atmosphere at 250°C to 600°C and 1 to 10 MPaG inside the reactor 111, the raw material mixture gas gets reacted and becomes a produced gas mixture containing various aromatic compounds. The produced gas mixture thus obtained is cooled to around normal temperature and introduced into a gas-liquid-liquid separator (not illustrated). The produced gas mixture, containing condensed high-boiling components, is separated into three layers, namely, a water phase (lower layer) containing water-soluble components, an oil phase (middle layer) containing an aromatic compound mixture, and a gas phase (upper layer) containing unreacted gases.

The oil phase forming the middle layer containing an aromatic compound mixture among the three layers is combined with the aromatic compound mixture manufactured in the step B (step C), followed by separation into various aromatic compound products and purification thereof (step D). On the other hand, the gas phase forming the upper layer is mixed as a circulation gas into the flow of the raw material mixture gas on the entrance side of the reactor 111 while being partly purged as a purged gas to the outside of the system. Moreover, the water phase forming the lower layer is sent to and treated by a waste water treatment apparatus, and then discharged to the outside of the system.

### (Step B)

In Fig. 2, heavy naphtha 120 from which its sulfur components have been removed in advance is catalytically reformed in a reformer 121 filled with a reforming catalyst to thereby be converted into a mixture (reformate) containing various aromatic compounds.

### (Steps C and D)

In Fig. 2, the oil phase drawn out of the middle layer in the gas-liquid-liquid separator in the step A and the product (reformate) obtained by the catalytic reforming 22 of heavy naphtha in the step B are combined in the step C and then subjected to purification processes 122 such as separation, isomerization, and disproportionation in the step D to thereby be separated into individual aromatic compounds.

### (Utilization of Excess Hydrogen)

In Fig. 2, while the hydrogen generated in the process of manufacturing an aromatic compound mixture (reformate) by the catalytic reforming 121 of heavy naphtha in the step B is used in pre-processing of the heavy naphtha and the like, there is still a portion left unused, which is excess hydrogen. This is used in the step A as a raw material for synthesizing aromatic compounds from hydrogen and carbon dioxide. This enables effective utilization of the excess hydrogen.

### (Collection and Utilization of Carbon Dioxide from Combustion Exhaust Gas)

The carbon dioxide contained in the combustion exhaust gases from the heating furnaces in the steps B and D can be collected by absorptive separation 112 via amine absorption or the like, and this can be used as a raw material for the aromatic compound synthesis from hydrogen and carbon dioxide in the step A.

### (Material Balance)

In Fig. 2, each numerical character surrounded by a diamond and attached to a line representing the flow of a substance(s) indicates the corresponding stream's number. Table 1 is an example material balance in which the mass flow rate of each stream and the mass fraction of its component(s) in Example 2 are listed.

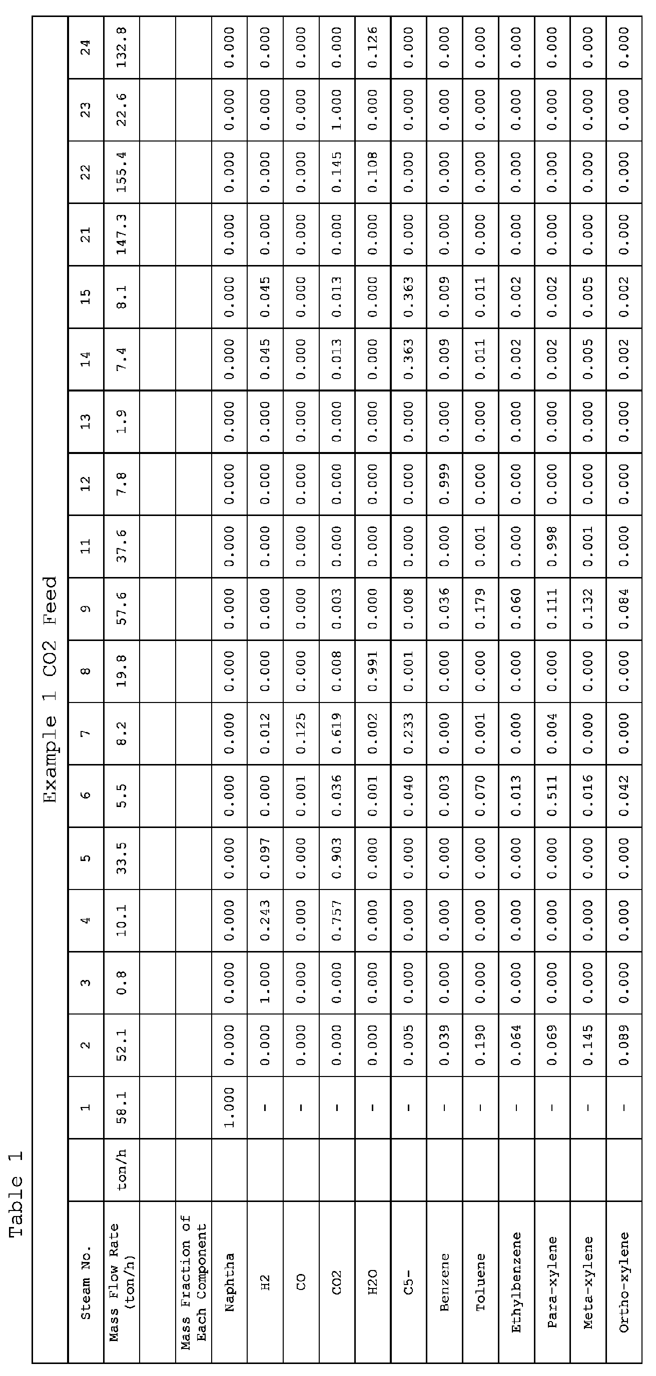

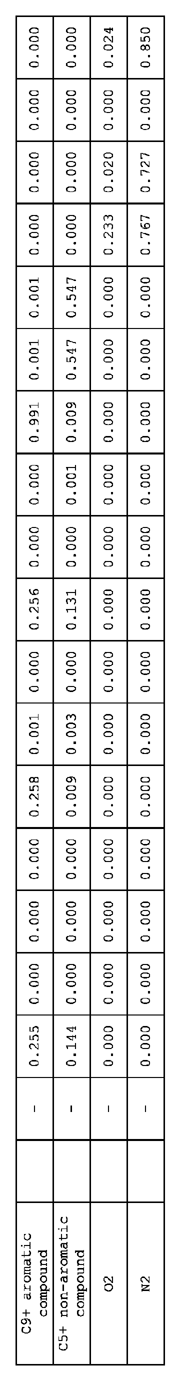

### Comparative Example

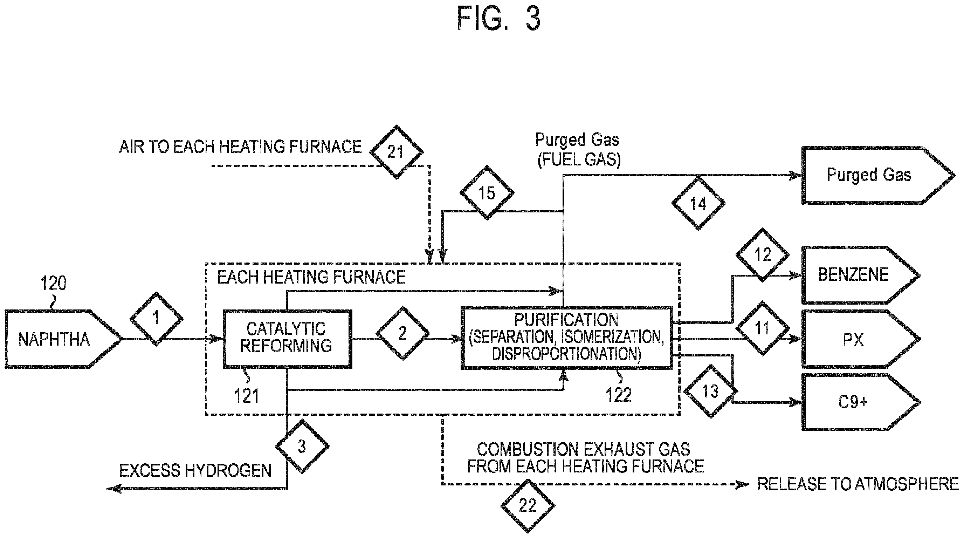

Fig. 3 illustrates a comparative example representing a case of excluding from Fig. 2 the step A (step of synthesizing aromatic compounds from hydrogen and carbon dioxide) and the step C (step of combining the aromatic compounds obtained in the step A and the aromatic compounds obtained in the step B). In other words, the comparative example illustrated in Fig. 3 is a process configuration including only the steps B and D in Example 2. In this comparative example, which is without the step A, the excess hydrogen generated in the step B and the carbon dioxide collected from the exhaust gas generated in the heating furnace in each step cannot be utilized as the raw materials in the step A.

### (Material Balance)

In Fig. 3, each numerical character surrounded by a diamond and attached to a line representing the flow of a substance(s) indicates the corresponding stream's number. Table 2 is an example material balance in which the mass flow rate of each stream and the mass fraction of its component(s) in this comparative example are listed.

**Table 2**

| Comparative Example | | | | | | | | | | |
|---|---|---|---|---|---|---|---|---|---|---|
| Steam No. | 1 | 2 | 3 | 11 | 12 | 13 | 14 | 15 | 21 | 22 |
| Mass Flow Rate (ton/h) | 64.5 | 57.9 | 0.9 | 37.6 | 8.3 | 1.1 | 8.6 | 8.0 | 147.2 | 155.2 |
| | | | | | | | | | | |
| Mass Fraction of Each Component | | | | | | | | | | |
| Naphtha | 1.000 | 0.000 | 0.000 | 0.000 | 0.000 | 0.000 | 0.000 | 0.000 | 0.000 | 0.000 |
| H2 | - | 0.000 | 1.000 | 0.000 | 0.000 | 0.000 | 0.047 | 0.047 | 0.000 | 0.000 |
| CO | - | 0.000 | 0.000 | 0.000 | 0.000 | 0.000 | 0.000 | 0.000 | 0.000 | 0.000 |
| CO2 | - | 0.000 | 0.000 | 0.000 | 0.000 | 0.000 | 0.000 | 0.000 | 0.000 | 0.145 |
| H2O | - | 0.000 | 0.000 | 0.000 | 0.000 | 0.000 | 0.000 | 0.000 | 0.000 | 0.108 |
| C5- | - | 0.005 | 0.000 | 0.000 | 0.000 | 0.000 | 0.354 | 0.354 | 0.000 | 0.000 |
| Benzene | - | 0.039 | 0.000 | 0.000 | 0.999 | 0.000 | 0.010 | 0.010 | 0.000 | 0.000 |
| Toluene | - | 0.190 | 0.000 | 0.002 | 0.000 | 0.000 | 0.011 | 0.011 | 0.000 | 0.000 |
| Ethylbenzene | - | 0.064 | 0.000 | 0.000 | 0.000 | 0.000 | 0.002 | 0.002 | 0.000 | 0.000 |
| Para-xylene | - | 0.069 | 0.000 | 0.997 | 0.000 | 0.000 | 0.002 | 0.002 | 0.000 | 0.000 |
| Meta-xylene | - | 0.145 | 0.000 | 0.001 | 0.000 | 0.000 | 0.006 | 0.006 | 0.000 | 0.000 |
| Ortho-xylene | - | 0.089 | 0.000 | 0.000 | 0.000 | 0.000 | 0.002 | 0.002 | 0.000 | 0.000 |
| C9+ aromatic compound | - | 0.255 | 0.000 | 0.000 | 0.000 | 0.983 | 0.001 | 0.001 | 0.000 | 0.000 |
| C5+ non-aromatic compound | - | 0.144 | 0.000 | 0.000 | 0.001 | 0.017 | 0.565 | 0.565 | 0.000 | 0.000 |
| O2 | - | 0.000 | 0.000 | 0.000 | 0.000 | 0.000 | 0.000 | 0.000 | 0.233 | 0.020 |
| N2 | - | 0.000 | 0.000 | 0.000 | 0.000 | 0.000 | 0.000 | 0.000 | 0.767 | 0.727 |

This application claims the benefit of priority from Japanese Patent Application No. 2021-120306, filed on July 21, 2021, the contents of which are incorporated by reference as a part of this application.

### Reference Signs List

10 raw material mixture gas (H₂/CO/CO₂)
11 aromatic compound synthesis reactor
12 gas-liquid-liquid separator
20 heavy naphtha (raw material for manufacturing reformate-derived aromatic components)
21 hydrogenation purification apparatus
22 catalytic reforming apparatus
30 naphtha cracking residue (raw material for manufacturing cracked petroleum-derived aromatic compounds)
31 hydrogenation purification apparatus
40 LPG/light naphtha (raw material for an aromatic compound synthesis process)
41 aromatic compound synthesis reaction apparatus
51 reformate splitter
52 aromatic compound extractive separation apparatus
53 distillation apparatuses (benzene tower/toluene tower)
54 dealkylation reaction apparatus
55 disproportionation reaction apparatus
56 distillation apparatus (xylene tower)
57 adsorption apparatus (para-xylene separation tower)
58 xylene isomerization reaction apparatus
59 distillation apparatus (heavy aromatic compound tower)
110 raw material mixture gas
111 aromatic compound synthesis reaction apparatus (including gas-liquid-liquid separation and gas phase circulation)
112 carbon dioxide separation-collection apparatus
120 naphtha (desulfurized)
121 catalytic reforming apparatus
122 purification (separation, isomerization, disproportionation) apparatus

## Claims

1. An aromatic compound manufacturing method **characterized in that** the method comprises:
a step A of manufacturing an aromatic compound mixture from a raw material mixture gas containing carbon dioxide or carbon monoxide or both of these and hydrogen;
a step B of manufacturing an aromatic compound mixture from a raw material containing a fossil resource- or biomass-derived hydrocarbon;
a step C of combining the aromatic compound mixture manufactured in the step A and the aromatic compound mixture manufactured in the step B; and
a step D of separating a desired aromatic compound from the aromatic compound mixture combined in the step C and purifying the desired aromatic compound.

2. The method according to claim 1, wherein the step A includes
a reaction step of reacting the raw material mixture gas with a reaction catalyst by bringing the raw material mixture gas into contact with the reaction catalyst under high temperature and high pressure, to thereby obtain a produced gas mixture containing an aromatic compound,
a separation step of separating the produced gas mixture obtained in the reaction step into a water phase containing a water-soluble component, an oil phase containing an aromatic compound mixture, and a gas phase containing an unreacted gas by cooling the produced gas mixture and thus condensing a high-boiling component therein, and
a circulation step of mixing at least a part of the gas phase separated in the separation step into the raw material mixture gas, and
the oil phase separated in the separation step is combined with the aromatic compound mixture manufactured in the step B.

3. The method according to claim 2, wherein in the separation step, a gas-liquid mixture obtained by cooling the produced gas mixture is firstly separated into a liquid phase and the gas phase, and then the separated liquid phase is separated into the oil phase and the water phase by a separation method utilizing a difference in relative density.

4. The method according to claim 2 or 3, wherein in the circulation step, a part of the gas phase to be circulated is purged, and hydrogen separated and collected from the purged gas is mixed into the raw material mixture gas.

5. The method according to claim 4, wherein pressure swing adsorption or membrane separation using a hydrogen separation membrane is performed as a method of separating and collecting hydrogen from the purged gas in the circulation step.

6. The method according to claim 2 or 3, wherein in the circulation step, a part of the gas phase to be circulated is purged, and the purged gas is used as a part of a fuel for a heating furnace in the step B or D.

7. The method according to any one of claims 2 to 6, wherein in the step B or D, carbon dioxide is separated and collected from a combustion gas from a heating furnace, and the collected carbon dioxide is mixed into the raw material mixture gas in the step A.

8. The method according to any one of claims 2 to 7, wherein the reaction catalyst used in the reaction step is a mixed catalyst containing a mixture of a first catalyst containing an oxide of at least one metal selected from among chromium, zinc, and copper and a second catalyst containing a ZSM-5-type zeolite.

9. The method according to claim 8, wherein in the reaction step, the raw material mixture gas is brought into contact with the reaction catalyst at a reaction temperature of 250 to 600°C and a reaction pressure of 1 to 10 MPaG.

10. The method according to any one of claims 2 to 9, wherein the raw material mixture gas and the produced gas mixture are caused to exchange heat with each other, and then the raw material mixture gas is transferred to the reaction step.

11. The method according to any one of claims 1 to 10, wherein
the step B includes a step of catalytically reforming heavy naphtha, and
excess hydrogen generated in the catalytic reforming step is used as at least a part of the hydrogen forming the raw material mixture gas in the step A.

12. The method according to any one of claims 1 to 11, wherein
the step B includes a step of aromatizing light naphtha, LPG, or an olefin, and
excess hydrogen generated in the aromatization step is used as at least a part of the hydrogen forming the raw material mixture gas in the step A.

13. The method according to any one of claims 1 to 12, wherein hydrogen produced by electrolysis of water using electric power generated by photovoltaic, wind, water, geothermal, biomass, or nuclear power generation is used as at least a part of the hydrogen forming the raw material mixture gas in the step A.

14. The method according to any one of claims 1 to 13, wherein carbon dioxide separated from a combustion exhaust gas from a thermal power plant or a heating furnace, carbon dioxide separated in an ammonia manufacturing apparatus, an ethylene glycol manufacturing apparatus, or a hydrogen manufacturing apparatus, carbon dioxide separated from a gas produced by a coal, biomass, or waste gasification furnace, carbon dioxide separated from a blast furnace at an ironworks, or carbon dioxide separated from atmospheric air is used as at least a part of the carbon dioxide forming the raw material mixture gas in the step A.

15. The method according to any one of claims 1 to 14, wherein a synthetic gas produced by a gasification furnace, an off-gas discharged from a blast furnace at an ironworks, an off-gas separated in a hydrogen manufacturing apparatus, a synthetic gas produced by co-electrolysis of water and carbon dioxide, or a synthetic gas produced by a reverse shift reaction of hydrogen and carbon dioxide is used as at least a part of the raw material mixture gas in the step A.
